# EUROPEAN PATENT APPLICATION

(11) **EP 4 116 400 A1**
(43) Date of publication of application: **11.01.2023**
(21) Application number: 21184909.6
(22) Date of filing: 09.07.2021
(51) Int. Cl.: C12M 1/12, C12M 1/00, C12M 1/34

(54) **MODULE FOR INCUBATION AND PERFUSION OF BIOLOGICAL MATERIALS**

(71) Applicant: Dumas, Dominique, 54000 Nancy (FR)
(72) Inventor: Dumas, Dominique, 54000 Nancy (FR)
(74) Representative: Icosa Europe

(57) **Abstract**

The present invention relates to a module (10) for incubation of a biological material, the module (10) comprising a body (101) having an inlet (102) and an outlet (103) for supplying and evacuating a liquid medium to the module (10), the body (101) comprising: an internal space for accommodating at least one removable incubating chamber (11) configured to contain the biological material and the liquid medium, and a perfusion system configured to convey the liquid medium between the inlet and the outlet through the removable incubating chamber (11), the perfusion system comprising a first port (122) to supply the liquid medium in the chamber and a second port (123) to evacuate the liquid medium from the chamber, each nozzle being configured to be located above two opposite sides of the chamber (11) and separated by a distance along a flow direction (x), wherein the module (10) is optically transparent at least along a vertical direction (z) perpendicular to the flow direction (x) so as to permit observation of the incubating chamber (11), and the perfusion system is at least partially embedded within walls of the body (101).

## Description

### FIELD OF INVENTION

The present invention relates to the field of incubation of biological materials.

More precisely, the invention relates to a module for incubation of a biological material, the module comprising a body having an inlet and an outlet for supplying and evacuating a liquid medium, respectively.

The invention further concerns a corresponding kit, a system, and a method.

### BACKGROUND OF INVENTION

Incubators are devices configured to maintain environmental conditions suitable for growing and maintaining biological materials such as living cells or organs. The most commonly used incubators may accommodate several incubating chambers containing different biological materials.

Different sizes of incubator exist, from benchtop incubators to warm rooms, none of which is suitable for specimen observation without altering the environmental conditions.

Indeed, the observation (e.g., visual inspection or observation by means of an imaging system) of the biological material requires its removal from the incubator. Therefore, the material is out of the controlled environment of the incubator for the time of observation and/or the time for transporting it to an imaging system.

Moreover, the removal of the material from the incubator, and its transport, may cause spilling of liquids bathing the biological material, thereby producing a risk of contamination.

Stage top incubators are incubators of compact dimensions, designed for live cell imaging with standard microscopes. These devices allow to maintain some environmental conditions (e.g., temperature, O₂, CO₂) for the cells cultured inside a chamber during the imaging experiment. However, the formation of water condensation in the chamber often occurs, thereby obstructing the view of the specimen and affecting the quality of the images. In addition to the image quality, condensation also affects the interpretation of results as it is a source of variability in the experimental conditions (e.g., volume reduction, alteration of the concentration of supplements due to evaporation, nefarious effects on the biological material caused by the fall of water droplets).

Stage top incubators with heated lids may partially reduce the condensation; however, the lid must be heated at a temperature higher than the bottom temperature, thereby creating a temperature uniformity across the biological material. Moreover, they do not completely eliminate water evaporation, thence condensation, and they do not allow to perform a thorough observation of the material. Indeed, each stage top incubator (with or without heated lid) is designed for being used with a particular microscope type (usually, an inverted microscope), and is not adapted for observation of the biological material with other imaging techniques.

Typically, heated lids obstruct the passage of light through the lid. Therefore, even in absence of condensation, it would still not be possible to inspect the biological material through the heated lid of a stage incubator.

The observation of biological materials with stage top incubators is also time consuming as a large number of preliminary operations and waiting steps are required, such as the connection of gas or liquid lines into the chamber containing the sample, and the waiting time for reaching stable environmental conditions inside the chamber.

Moreover, neither conventional incubators nor top stage incubators allow to individually control the environmental conditions of a sample. In other words, the environmental conditions are the same for all the biological materials inside the incubator.

The present invention aims to solve the above-mentioned problems in providing an incubation module which is versatile and allows a thorough observation of the biological material therein contained, without disruption of its environmental conditions.

The current invention further aims at providing a leak-proof module requiring a limited number of manual operations, thus safer and easier to transport with respect to the incubators of the state of the art.

It is also a purpose of the present invention to provide an incubator which allows to create homogeneous or heterogeneous environmental conditions as needed.

### SUMMARY

To this end, the present invention relates to a module of the aforementioned type for incubation of a biological material, the module comprising a body having an inlet and an outlet for supplying and evacuating a liquid medium to the module, the body comprising:
i. an internal space for accommodating at least one removable incubating chamber configured to contain the biological material and the liquid medium,
ii. a perfusion system configured to convey the liquid medium between the inlet and the outlet through the removable incubating chamber, the perfusion system comprising a first fluidic port to supply the liquid medium in the chamber and a second fluidic port to evacuate the liquid medium from the chamber, each nozzle being configured to be located above the chamber and separated by a distance along a flow direction,

wherein the module is optically transparent at least along a vertical direction perpendicular to the flow direction so as to permit observation of the incubating chamber, and the perfusion system is at least partially embedded within walls of the body.

Indeed, the location of the ports of the perfusion system above the chamber allows to create a flow of the liquid medium along a flow direction which is right under the lid of the incubating chamber. The evaporation of water, and thence water condensation, in this area is thus avoided. Therefore, the module allows to inspect the biological material with different modalities (e.g., macroscopy, microscopy, digital holographic microscopy and the like) and obtain images with high-quality and resolution.

Moreover, as the module is optically transparent along a vertical direction, it allows to observe the biological material from different angles. For instance, the same biological material may be observed both from the top (for instance via an upright microscope) and from the bottom (for instance via an inverted microscope).

As the perfusion system is at least partially embedded within walls of the body, there is no need to manually insert tubes for supplying the liquid medium inside the chamber. Therefore, the module further allows to reduce the manual operations, which ultimately results in a more versatile and safer system.

In a preferred embodiment, the perfusion system is totally embedded within the walls of the body, and does not comprise any additional hose or tube (except to connect the inlet of the module to a pump).

In one embodiment, each port comprises a flow distributor configured to distribute the flow of liquid medium in a direction transverse to the flow direction in the at least one incubating chamber.

The flow distributor divides the flow of the liquid medium, such that the flow of liquid medium is better distributed in the chamber.

In a preferred embodiment, the flow distributor extends along a whole vertical wall of the chamber, such that it covers the whole underlid of the chamber. The formation of water condensation is thus hindered on the whole chamber.

Moreover, the flow distributor allows to better control the shear stress on the biological material. A predetermined shear stress may be applied for instance to simulate specific laminar flow conditions observed *in vivo.* For instance, a shear stress in the ranges of about 1―50 dyn/cm2 may be selected for a biological material comprising endothelial cells, as this range is usually found in blood vessels. Different shear stress may be selected for different biological materials and/or application.

In one embodiment, the body comprises a top face comprising at least one transparent portion and a bottom face comprising an aperture opening into the internal space (104) and configured to receive the at least one incubating chamber, said aperture being located below the ports along the vertical direction.

Advantageously, this embodiment allows to insert the module in a downward direction on top of the chamber, the latter can thus be received inside the internal space, via the aperture, without the need to touch the chamber. This allows to reduce the risk of contamination and the risk of damaging or breaking the chamber.

The module according to the invention may comprise one or several of the following features, taken separately from each other or combined with each other:
- each flow distributor comprises a recess or groove in the transparent portion of the top face opening into the internal space;
- a sealing element for providing leak-tightness between the incubating chamber and the body, the sealing element preferably being a flat gasket surrounding the internal space;
- the perfusion system further comprises a non-return valve, which allows to prevent backflow of the liquid medium;
- a heating element configured to be at a controlled temperature, the heating element preferably comprising two heating ribbons on two opposite sides of the body, which allows to maintain a controlled temperature inside the chamber;
- a power supply, which is particularly advantageous for maintaining the environmental conditions inside the chamber during transportation or in case of power failure;
- at least one sensor for sensing a parameter relating to the environment inside the at least one incubating chamber (for example, the parameter may be O₂, CO₂, N₂, temperature, etc.);
- the body is at least partially made up of glass, methyl methacrylate, plastic polymers, or polyoxymethylene; advantageously, these materials are compatible with magnetic resonance machines.

The present invention also relates to a kit comprising a module according to any one of the embodiments described hereinabove, and a microscope stage, the microscope stage comprising at least one slot configured to receive the module. This embodiment is advantageously adapted for microscopy observation of the biological material.

The present invention also relates to a system for incubation of a biological material, the system comprising at least one module according to any one of the embodiments described hereinabove and a controller, wherein the controller comprises at least one pump configured to pump the liquid medium from a reservoir to the inlet of the module.

In one embodiment, the system comprises at least two modules connected in series or in parallel.

Advantageously, this embodiment allows to mimic and observe various biological environments comprising different biological materials communicating with each other in a parallel or serial manner, such as microenvironmental niches, cell-cell interactions, cell signaling such as paracrine signaling, and the like.

The present invention also relates to a method for incubating a biological material in a module according to any one of the embodiments described hereinabove, the method comprising a step of conveying the liquid medium through the at least one incubating chamber via the perfusion system.

In one embodiment, the liquid medium is conveyed at a continuous flow rate.

A continuous flow rate is preferable for most biological materials.

In one embodiment, the liquid medium is conveyed at a pulsatile flow rate.

A pulsatile flow rate is particularly advantageous for perfusion of biological materials such as endothelial cells or cardiac cells (cardiomyocytes), or for cellular differentiation.

### BRIEF DESCRIPTION OF THE DRAWINGS

The invention will be better understood, and other aims, details, characteristics and advantages thereof will emerge more clearly on reading the detailed description which follows, of one or several embodiments of the invention given by way of illustration. The drawings are not drawn to scale and are not intended to limit the scope of the claims to the embodiments depicted.

On these drawings:
- **Figure 1** is a perspective view showing a module 10 according to a first embodiment;
- **Figure 2** is an exploded view of the module 10 of **figure** 1.
- **Figure 3A** is a schematic top view of a module 10 compliant with the present disclosure;
- **Figure 3B** is a schematic lateral view of the module 10 of **figure 3A****;**
- **Figure 4** is a cross-sectional perspective of the top face 15 of the module 10 of **figure 1****;**
- **Figure 5** is an exploded view of a module 10 comprising a sealing element 14;
- **Figure 6** is a phantom perspective of an exemplary module 10;
- **Figure 7** is a cross-sectional perspective of the module 10 of **figure 6****;**
- **Figure 8** is a schematic diagram illustrating the flow of the liquid medium in the perfusion system 12 of the module 10 of **figures 6****;**
- **Figure 9** is a cross-sectional perspective of the module 10 of **figure 5****;**
- **Figure 10** is an exploded view of the module 10 of **figure 5****.**
- **Figure 11A** is a schematic top view of a kit 2 compliant with the present disclosure;
- **Figure 11B** is a schematic lateral view of the kit 2 of **figure 11A****;**
- **Figure 12** is a perspective view of an exemplary controller 13 of a system 1 compliant with the present disclosure; and
- **Figure 13** is a diagram schematically illustrating a system 1 comprising two controllers 13.

### DETAILED DESCRIPTION

This invention relates to a module 10 for incubation of a biological material.

By *"biological material"* it is meant any living material requiring controlled environmental conditions for growth and survival. Biological materials comprise, without limitations, cells, microorganisms, tissues, small organs, small animals, organoids and the like.

### The body 101

The module 10 of the invention is configured to accommodate at least one removable incubating chamber 11 containing the biological material. The body 101 may comprise several subparts assembled together or be formed as a single monolithic part.

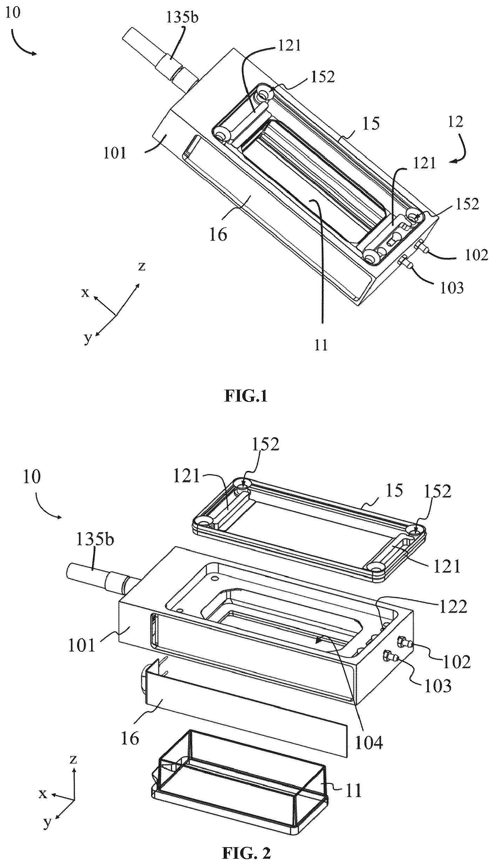

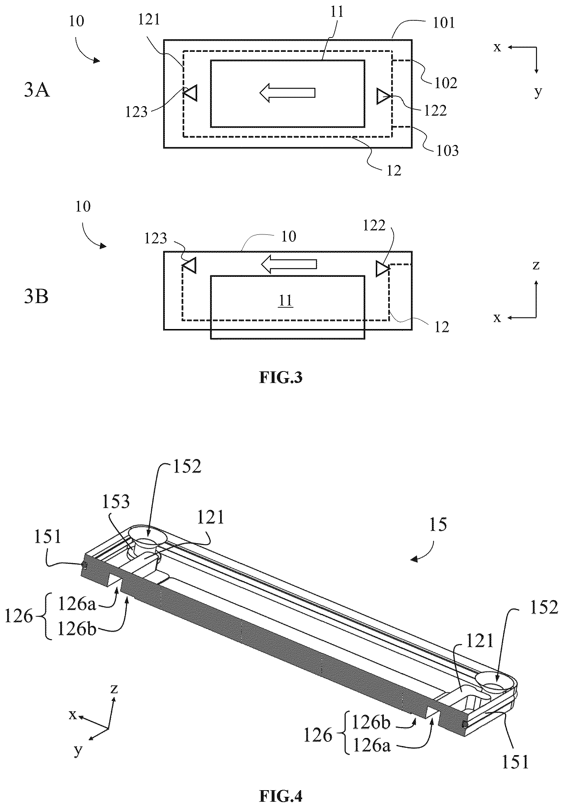

As can be seen on **figure 1****,** the module 10 comprises a body 101 which has at least one inlet 102 and at least one outlet 103. The inlet 102 and outlet 103 respectively allow to supply and evacuate a liquid medium to the module 10. More precisely, the module 10 comprises a perfusion system 12 (figure 3) configured to convey the liquid medium between the inlet 102 and the outlet 103 through the removable incubating chamber 11.

In one particular embodiment, the module 10 is entirely made of non-magnetic materials such as plastic polymers. Advantageously, this embodiment allows to introduce the module 10 inside an MRI machine to acquire magnetic resonance images and/or magnetic resonance spectra of the biological materials.

In this case, the body 101 is preferably made of polyoxymethylene, so as to further ensure good mechanical properties.

### The perfusion system 12

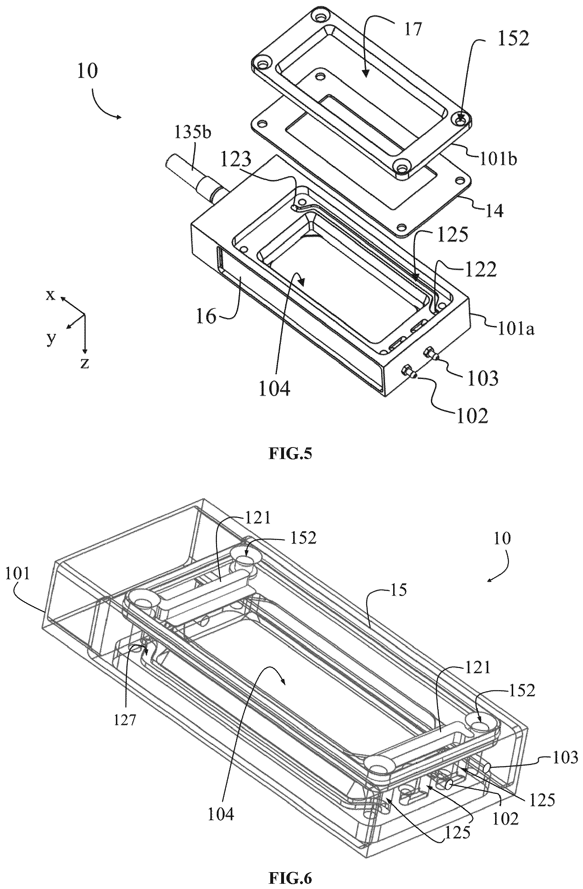

The perfusion system 12 is at least partially embedded within walls of the body 101. Preferably, it is entirely embedded in the body 101 (as in figure 6). For instance, the perfusion system 12 may comprise a series of communicating channels 125 (figures 5 and 6), in the form of grooves or recesses, inside the body 101 of the module which connect the inlet 102 and the outlet 103.

Having the perfusion system 12 partially or entirely embedded in the body 101 allows to provide a module 10 which is ready-to-use once and adapted to perfuse liquid medium inside any chamber which might be accommodated in the internal space 104, *i.e.,* without the need to customize the perfusion system 12 to a predetermined type of chamber 11. Preferably, the perfusion system 12 is configured to fill the whole internal space 104 including the incubating chamber 11 with the liquid medium.

The perfusion system 12 is advantageously suited to perfuse various types of liquid medium through the chamber 11 depending on the particular application for which the module 10 is employed and/or the type of biological material. For instance, it may be adapted to convey cell culture media, saline solutions, buffers, trypsin solutions (or similar solutions for dissociation of cell monolayers and/or digestion of tissues), disinfectants (such as ethanol-containing solutions), paraformaldehyde or other fixation solutions, detergents or permeabilizing agents, protein-blocking solution, cryoprotectants, fluorescent or non-fluorescent dyes, and the like. As aforementioned, in one embodiment the module 10 is made of a magnetic resonance compatible material. In this case, the perfusion system 12 may be configured for instance to convey hyperpolarized substrates, so as to observe their metabolic conversion in real-time.

Notably, the perfusion of media such as saline buffers and trypsin solutions is particularly advantageous for alimenting automatically or manually feed a 3D tissue printer by connecting the perfusion system 12 to different modules 10.

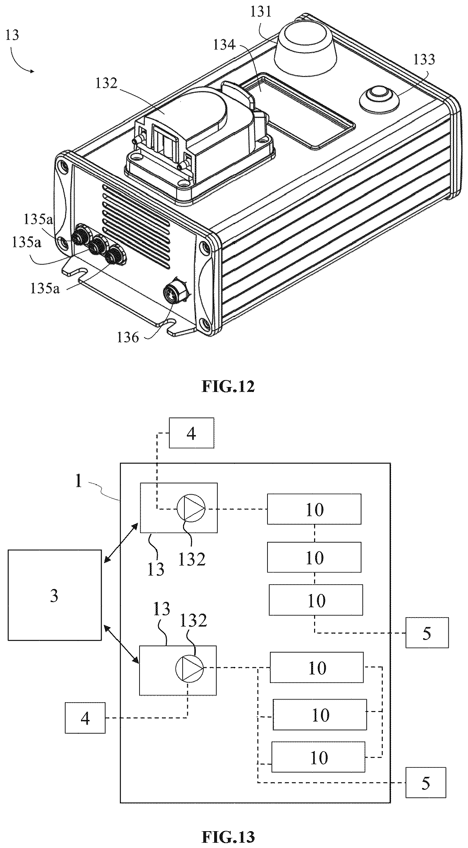

Accordingly, the inlet 102 and outlet 103 may be adapted to be respectively connected to a fluid reservoir 4 (figure 13) containing the liquid medium to be supplied, and a fluid reservoir 5 for collecting the evacuated medium and, in some cases, waste products which may be released or detached from the biological material. In this case, the inlet 102 and outlet 103 may be adapted to be connected to said reservoirs 4, 5 via tubes, Luer Lock connections or any standard connection type.

### The top face 15

Moreover, the module 10 may comprise a top face 15. In this case, some portions of the perfusion system 12 may be located in the top face 15.

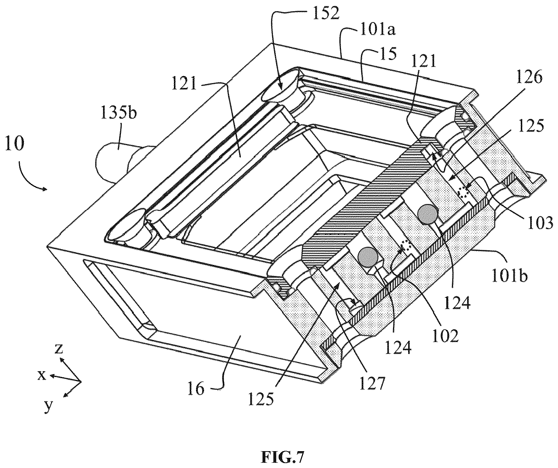

For instance, in the module of **figure 1****,** the top face is transparent and it accommodates two flow distributors 121. The flow distributor 121 allow to distribute the flow of liquid medium in a direction y which is transverse to the flow direction x (figure 7). Each of said flow distributors 121 is in fluidic communication with the inlet 102 or the outlet 103 via the perfusion system 12, for instance via one or more channels 125 inside the body 101 (figure 5 and figure 6 showing examples of said channels 125).

Moreover, the top face 15 may comprise fixation means configured to secure the top face 15 against the body 101. For instance, it may comprise one or more running through holes 152 configured to house respective fastening screws (screws not shown).

In the example of **figure 1****,** the module 10 further comprises a control port 135b adapted to be connected to a corresponding port 135a of a controller 13 (figure 12).

### The heating element 16

Advantageously, the module 10 may also comprise a heating element 16.

The heating element 16 may be for instance a resistance, a low voltage heating element, a Peltier cell, an amorphous metal ribbon, or any suitable heating element adapted to be maintained at a controlled temperature.

For instance, the heating element 16 may be a metallic film. In this case, the module 10 may comprise a printed circuit board comprising a heating module operatively connected to the film.

### The internal space and the aperture 17

As aforementioned, the body 101 of the module 10 is configured to accommodate a removable incubating chamber 11. More precisely, the body 101 comprises an internal space 104 (figure 2) configured to receive said chamber 11.

The incubating chamber 11 may be easily inserted and removed from the internal space 104 of the module, and eventually replaced with a different one as needed. The module 10 is therefore versatile as it is adapted to receive different incubating chambers 11, and there is no need to customize the perfusion system (or other elements of the module 10) to a predetermined type of chamber 11.

The incubating chamber 11 might be for instance a Petri dish, a chamber slide (or chambered coverslip), a multi-well plate, and the like.

**Figure 2** illustrates an exploded view of a module 10 according to the disclosure. In this example, the internal space 104 is adapted to accommodate a 1 well chamber slide 11.

For instance, a portion of the body 101 may comprise an aperture 17 (figure 5) opening into the internal space 104 and configured to receive the at least one incubating chamber 11. Said aperture 17 may be located on a bottom face 101b (figures 5, 7 and 9) of the body 101. Advantageously, this embodiment allows to insert the module 10 in a downward direction on top of the incubating chamber 11 until the incubating chamber 11 is inside the internal space 104. In this case, the body 101 may further comprise means for holding the incubating chamber 11 inside the internal space 104 after the insertion. Such means may advantageously comprise a flat sealing gasket 14 (figure 5), as will be described later.

The body 101 may further comprise means for improving the position of the assembly *(i.e.,* the chamber 11 and the module 10) such as wedges or machined patterns in the body 101. This embodiment ensures the parallelism between the chamber 11 and the module 10.

Alternatively, the chamber 11 may be retained inside the chamber 11 via snapfitting means, spring loaded clips, or any other means suitable for holding the chamber in place.

In some embodiments, the entire chamber 11 may be accommodated in the internal space 104.

Alternatively, the internal space 104 may be configured to accommodate a portion of the chamber 11.

**Figure 3** illustrates an exemplary module having an internal space 104 configured to accommodate a portion of the chamber 11. More precisely, **figure 3A** and **figure 3B** respectively illustrates a schematic top view and a schematic lateral view of a module 10 compliant with the present disclosure. As can be seen on **figure 3B**, the chamber 11 protrudes outwardly of the body 101.

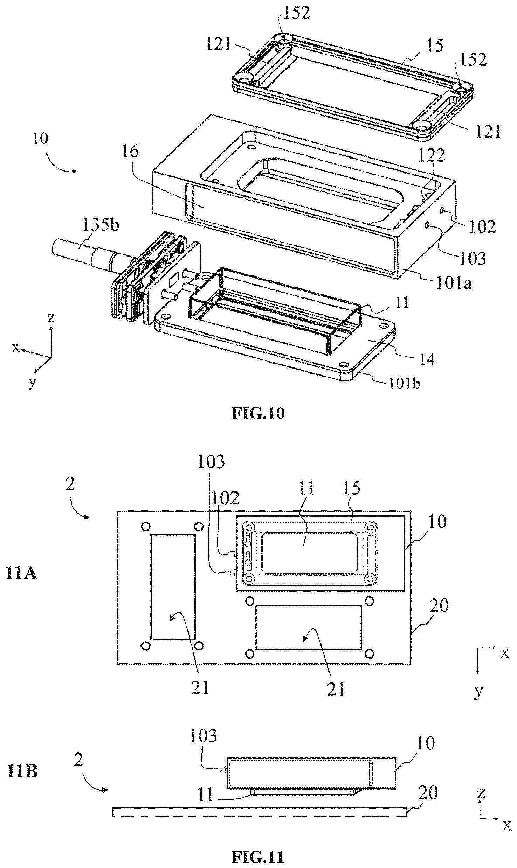

Advantageously, when the chamber 11 is not entirely comprised in the internal space 104, its removal is easier. Moreover, it is possible to place the module 10 on top of a microscope stage 20 (figure 10), so that the portion of the chamber 11 which protrudes outwardly of the internal space 104 is fitted inside a slot of the microscope stage 20.

### The fluidic ports 122,123

The perfusion system 12 schematically illustrated in **figure 3** also comprises a first fluidic port 122 (or arrival port) configured to supply the chamber 11 with liquid medium and a second fluidic port 123 (or exit port) configured to evacuate the liquid medium, each port 122, 123 being configured to be respectively located downstream of the inlet 102 and upstream of the outlet 103 along a flow direction x (represented in **figure 3** by a white arrow).

In this example, the first and second fluidic ports 122, 123 are respectively located above two opposite sides of the chamber 11 along a flow direction x. In this case, the liquid medium may be supplied on one side of the incubating chamber by means of the first port 122, and evacuated at the opposite side of the incubating chamber 11 by means of the second port 123.

Alternatively, the first and second fluidic ports 122, 123 may be located on the same side of the chamber, and be separated by a distance along the flow direction x.

Advantageously, having the first and second ports 122 located above the removable chamber 11 avoids the formation of water condensation on the portion of the chamber lid which is between the first port 122 and the second port 123, as this portion is irrorated by the liquid medium.

Moreover, each fluidic port 122, 123 may advantageously comprise a flow distributor 121 configured to distribute the flow of the liquid medium.

### The flow distributor 121

**Figure 4** illustrates a cross sectional view of a top face 15 comprising a flow distributor 121 configured to distribute the flow of liquid medium in a direction y transverse to the flow direction x.

In this particular example, the flow distributor 121 comprises a recess 126 in the top face 15 which opens into the internal space 104. More precisely, the recess 126 is a stepped recess with a deeper portion 126a in communication with one of the first or second port 122 or 123, and a shallower portion 126b opening into the internal space 104. In this example, the top face 15 is transparent. In a variant, only a portion of the top face is transparent 15. In this case, the flow distributor 121 is preferably comprised in the transparent portion of the top face 15.

The transparent portion, or the entire top face 15, may be made for instance of glass, methyl methacrylate, or any suitable transparent material.

### Sealing elements on the top face 151, 153

The top face 15 of **figure 4** also comprises a gasket rim 151 forming a closed loop around the perimeter of the top face 15, which allows to provide leak-tightness between the top face 15 and the body 101 when the two are assembled together.

As aforementioned, the top face 15 may comprise running through holes 152 configured to house fastening screws. In this case, further sealing elements, for instance gasket rings 153, may be provided around the holes 152 in the top face 15, which contribute to the sealing.

**Figure 5** illustrates a module 10 according to the invention in an upside-down position. In this case, the body 101 of the module comprises a central portion 101a and a bottom face 101b comprising an aperture 17 opening into the internal space 104.

The bottom face 101b may also comprise running through holes 152 communicating with the holes 152 in the top face 15 (figure 4), thereby allowing to fasten together the top face 15, the central portion 101a and the bottom face 101b of the body 101 via fastening screws.

However, in some embodiments said portions or faces 15, 101a, 101b may be fixed together with adhesive or other fixing means. Alternatively, they may be integrally formed.

### The channels 125

**Figure 6** is a phantom view showing a module 10 in which the perfusion system 12 is entirely embedded within the body 101.

This perfusion system 12 comprises several channels 125 which put in communication the recesses 126 in the top face 15 and the grooves 127 in the body 101 with the inlet 102 and the outlet 103.

Said channels 125 are better shown in **figure 7**.

More precisely, **figure 7** is a a cross-sectional perspective of the module 10. This module 10 comprises a vertical channel 125 (visible on the left side of the central portion 101a of the body 101) configured to connect a groove 127 in the central portion 101a of the body 101 with the inlet 102, so as to allow the liquid medium to circulate in said groove 127 and reach the flow distributor 121.

Similarly, a second vertical channel 125 (visible on the right side of the central portion 101a of the body 101) is configured to connect a recess 126 in the top face 15 with the outlet 103, so as to put in communication the flow distributor 121 with the outlet 103, and evacuate the liquid medium.

### The valve 124

In this particular example, the perfusion system 12 further comprises a non-return valve 124.

Advantageously, the non-return valve 124 allows to control the flow direction of the liquid medium and prevent backflow.

Moreover, said non-return valve 124 may be configured to ensure at least two distinct flow directions. This embodiment allows to provide different flow directions for different liquid media, without mixing them.

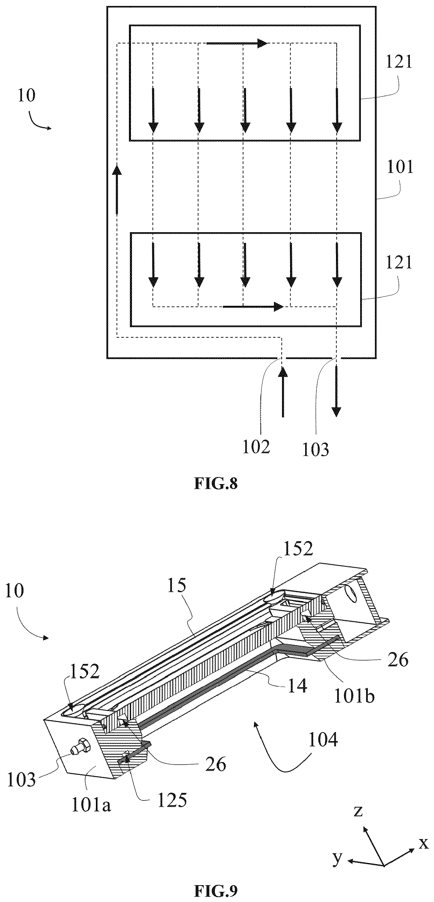

One flow direction is illustrated in **figure 8****.**

More precisely, **figure 8** illustrates a schematic top view of a module 10 in which the liquid medium supplied via the inlet 102 is conveyed via the channels 125 of the perfusion systems 12 (figures 5, 6 and 7 illustrate examples of said channels) to a fluidic port 122 comprising a flow distributor 121. The latter divides the flow of the liquid medium, in order to better distribute said liquid inside in the chamber 11. The liquid medium then reaches a second fluidic port 123 comprising a flow distributor 121 fluidly connected with the outlet 103, thence is evacuated.

### The sealing element 14

As aforementioned, the top face 15 may comprise one or more sealing elements such as gasket rims 151 and/or gasket rings 153 (figure 4) for providing leak-tightness between said top face 15 and the body 101 of the module 10.

Moreover, the module 10 may comprise another sealing element 14 so as to provide leak-tightness between the incubating chamber 11 and the body 101 when the chamber 11 is inside the internal space 104. This embodiment advantageously allows to insert or remove the chamber 11 in the module without leakage of the liquid possibly contained in the perfusion system 12.

As shown in **figure 9****,** said sealing element 14 may be for instance a flat gasket at least partially protruding towards the internal space 104. In this case, the sealing element 14 has a twofold function as it allows to retain the chamber 11 in place inside the internal space 104, and to avoid leaking of the liquid possibly present in the perfusion system 12 when the chamber 11 is removed.

More precisely, in the example of **figure 9****,** the body 101 of the module comprises a top face 15, a central portion 101a and a bottom face 101b, and the sealing element 14 is configured to be sandwiched between the central portion 101a and the bottom face 101b.

The sealing element 14 is preferably made of an elastomeric material, so as to ensure the sealing by deformation of the sealing element 14 caused by the pressure exerted by the chamber 11.

Of note, the incubating chambers 11 known in the art consist of a bottom portion for containing the biological material and a cover.

The sealing element 14 may advantageously be configured to seal the cover of the incubating chamber 11 against the bottom portion. One example of this embodiment is shown for instance in **figure 10****.**

In this case, the sealing element 14 provide additional leak-tightness between the bottom portion and the cover of the chamber 11. This is particularly advantageous for transporting the module 10, as the presence of this sealing element 14 allows to move, *e.g.* displace horizontally or vertically, or rotate in any direction, the module 10 without leakage of the content of the chamber 11.

Advantageously, the body 101 may comprise lips for gripping the sealing element 14. This allows to avoid the entrance of air in the internal space 104 of the body, which would jeopardize the sterility of the biological material on one hand and the maintenance of the internal pressure, i.e., the pressure inside the internal space 104, on the other hand.

### The power supply

The module 10 of the present disclosure may also comprise a power supply, for instance a battery. The power supply allows to provide an autonomous module 10 and it is particularly advantageous for maintaining the environmental conditions inside the chamber 11 for instance during transportation or in case of power failure.

### The sensors

The module 10 may further comprise sensors for directly or indirectly measuring the environmental conditions in the chamber 11.

Examples of said sensors comprise temperature sensor, capnometers, oxygen sensor. In some embodiments, the module 10 may also comprise fluorescence or phosphorescence sensors.

### The kit 2

The present invention also relates to a kit 2 comprising a module 10 according to any one of the embodiments presented hereinabove, and a microscope stage 20.

The stage 20 may be a manual stage or a motorized stage, such as a linear translation stages or a rotary stage.

The kit 2 according to the present disclosure may comprise one or more slots, each slot 21 being configured to receive one module 10. Preferably, the slots 21 are configured to receive a portion of the chamber 11 protruding outside of the internal space 104 of the module 10.

In this case, the stage 20 may be machined so that the protruding portion of the chamber 11 (for instance the slide of a chambered coverslip) is aligned with the surface of the stage 20 configured to face a microscope lens. Advantageously, this avoids the loss of focus (within the depth of field) during the movement of the lens.

In the example of **figure 11****,** the stage 20 of the kit 2 comprises three slots 21, one of which is accommodating a module 10. More precisely, in this example one slot 21 has a longer dimension which extends parallel to the y axis, and the other two slots 21 have a longer dimension which extends parallel to the x axis. This embodiment is particularly advantageous for motorized rotary microscope stages 20, because in this case a rotation smaller than 360° is sufficient to provide positioning of all the samples.

As can be seen in **figure 11****,** the stage 20 may comprise some magnetized areas around each slot 21, for holding the module 10 in place.

### The system 1

The present invention also relates to a system 1 for incubation of a biological material, said system 1 comprising at least one module 10 according to any one of the embodiments presented hereinabove and a controller 13.

As shown in **figure 12****,** the controller 13 preferably comprises at least one pump 132, for instance a peristaltic pump, configured to pump the liquid medium from a reservoir to the inlet 102 of the module 10. Preferably, the pump 132 is adapted to generate a flow rate comprised between 0 ml/min and 23 ml/min.

The controller 13 may comprise one or more inputs 133 (for instance a knob, or a touchscreen) for selecting environmental parameters. Such environmental parameters may comprise temperature, pH, O₂ concentration, CO₂ concentration, flow rate, shear stress and the like.

Advantageously, the present system 1 allows to set specific environmental conditions inside the module 10, so as to simulate the phenomenon of interest *(e.g.,* cell differentiation, maturation, growth, senescence, death, hypoxia, normoxia, and the like), and to observe the effect of such parameters on the biological material with different modalities.

The controller 13 may advantageously allow to set equal or different environmental parameters inside each module 10 of the system 1. In this case, it also comprises an input 131 (a knob in the example of figure 12) for selecting the module 10 of interest.

As can be seen in **figure 13****,** the parameters may be set by a user via a user interface 3 interacting with the controller 13. For instance, the controller may comprise a port 136 adapted to be connected to a computer. The user interface 3 includes any means appropriate for entering or retrieving the parameters, notably visual, tactile and/or audio capacities that can encompass any or several of the following means as well known by a person skilled in the art: a screen, a keyboard, a trackball, a touchpad, a touchscreen, a loudspeaker, a voice recognition system.

In this example, the system 1 comprises two controllers 13. More precisely, a first controller 13 is operatively connected to three modules connected in series (top controller), and a second controller 13 is operatively connected to three modules connected in parallel (bottom controller). Moreover, in this example each controller comprises a pump 132 configured to be connected to a respective fluid reservoir 4 and waste reservoir 5.

The controller 13 may also comprise an output, for instance a display 134, for outputting the parameters set by the user and/or the parameters measured by the sensors. The output may provide error messages in case the difference between one or more set parameters and the respective measured parameters is above a predetermined threshold.

The controller 13 may further allow to automatically select the type of liquid medium conveyed via the perfusion system. Advantageously, this embodiment allows to select, and convey through the chamber 11, a succession of different liquid media so as to operate a specific protocol. For instance, a succession of a saline solution, a dissociating agent, and a cell growth medium containing protease inhibitors may be selected to perform a conventional cell subculturing protocol. In this case, the module 10 allows to provide continuous and fully automated supply of cells. This embodiment is particularly advantageous for high throughput applications such as bioprinting.

The system 1 may comprise two or more modules 10 which may be independently controlled by the controller 13. Accordingly, the controller 13 may comprise one or more ports 135a (figure 12), each port being configured to be connected to a corresponding port 135b of a module 10.

Advantageously, the at least two modules 10 of the system 1 may be connected in series. This embodiment advantageously allows to mimic microenvironmental niches. For instance, it allows to mimic and observe paracrine effects, to observe niches such as the tumor cells-stroma niche, the immune cells-microbiota niche and the like.

Alternatively, the at least two modules 10 may be connected in parallel. This embodiment advantageously allows to co-culture two or more biological materials in parallel. For instance, it is possible to feed a downstream module 10 with soluble elements produced in parallel by two or more upstream modules. Moreover, this configuration is particularly advantageous to perform pharmacological efficacy studies.

As aforementioned, the module 10 may comprise a sealing element 14 and, optionally, the body 101 may comprise lips for gripping said sealing element 14. This embodiment is particularly advantageous when two or more modules 10 are connected in series or parallel inside a system 1, because it allows to maintain a stable internal pressure *(i.e.,* a stable pressure in the internal space 104) while the liquid is supplied to the perfusion system 12 of each module 10 by the same pump 132.

### The method

The present invention also relates to a method for culturing a biological material in a module 10 in accordance with the present disclosure.

The method comprises a step of conveying the liquid medium through the incubating chamber 11 via the perfusion system 12.

More precisely, the liquid medium is introduced in the perfusion system 12 via the inlet 102. Then, the medium is conveyed through the incubating chamber 11 via the first port 122 of the perfusion system 12.

The medium is preferably conveyed at a flow rate ranging from 0 mL/min to 23 mL/min.

The method may comprise a step of distributing the liquid medium in the biological in the chamber 11. In this case, the medium may be distributed via a flow distributor 121.

The method may comprise a step of evacuating the medium from the incubating chamber 11 via an second port 123 fluidly connected to the outlet 103.

For instance, the liquid medium may be conveyed via the perfusion system 12 at a continuous flow rate. The flow rate value may be set via the controller 13.

Alternatively, the liquid medium may be conveyed at a pulsatile flow rate.

Moreover, the method may comprise periodically modifying one or more environmental parameters so as to provide variable environmental conditions inside the chamber 11.

For instance, the method may comprise setting, via a user interface 3 communicating with the controller 13, two or more O₂ concentrations (or partial pressure), and a respective time for each O₂ concentration. This embodiment allows to create intermittent hypoxia inside the chamber, which is particularly advantageous to mimic and observe conditions such as stroke, sleep apnea syndrome, hypoxic niches, environment of stem cells, carcinogenesis.

## Claims

1. A module (10) for incubation of a biological material, the module (10) comprising a body (101) having an inlet (102) and an outlet (103) for supplying and evacuating a liquid medium to the module (10), the body (101) comprising:
i. an internal space (104) for accommodating at least one removable incubating chamber (11) configured to contain the biological material and the liquid medium,
ii. a perfusion system (12) configured to convey the liquid medium between the inlet and the outlet through the removable incubating chamber (11), the perfusion system (12) comprising a first fluidic port (122) to supply the liquid medium in the chamber and a second fluidic port (123) to evacuate the liquid medium from the chamber, each nozzle being configured to be located above the chamber (11) and separated by a distance along a flow direction (x),
wherein the module (10) is optically transparent at least along a vertical direction (z) perpendicular to the flow direction (x) so as to permit observation of the incubating chamber (11), and the perfusion system (12) is at least partially embedded within walls of the body.

2. The module (10) of claim 1, wherein each port (122, 123) comprises a flow distributor (121) configured to distribute the flow of liquid medium in a direction (y) transverse to the flow direction (x) in the at least one incubating chamber (11).

3. The module (10) according to claim **1** or claim **2,** wherein the body (101) comprises a top face (15) comprising at least one transparent portion and a bottom face comprising an aperture (17) opening into the internal space (104) and configured to receive the at least one incubating chamber (11), said aperture (17) being located below the ports (122, 123) along the vertical direction (z).

4. The module (10) according to claim **3,** wherein each flow distributor (121) comprises a recess (126) or groove in the transparent portion of the top face (15) opening into the internal space (104).

5. The module (10) according to any one of claims **1** to **4,** further comprising a sealing element (14) for providing leak-tightness between the incubating chamber (11) and the body (101), preferably the sealing element (14) is a flat gasket surrounding the internal space (104).

6. The module (10) according to any one of claims 1 to 5, wherein the perfusion system (12) further comprises a non-return valve (124).

7. The module (10) according to any one of claims 1 to 6, further comprising a heating element (16) configured to be at a controlled temperature, preferably the heating element comprises two heating ribbons on two opposite sides of the body (101).

8. The module (10) according to any one of claims 1 to 7, further comprising a power supply.

9. The module (10) according to any one of claims **1** to **8,** further comprising at least one sensor for sensing a parameter relating to the environment inside the at least one incubating chamber (11).

10. The module (10) according to any one of claims **1** to **9,** wherein the body (101) is at least partially made up of glass, methyl methacrylate, plastic polymers, or polyoxymethylene.

11. A kit (2) comprising a module (10) according to any one of claims **1** to **10,** and a microscope stage (20), the microscope stage comprising at least one slot (21) configured to receive the module (10).

12. A system (1) for incubation of a biological material, the system (1) comprising at least one module (10) according to any one of claims **1** to **10** and a controller (13), wherein the controller (13) comprises at least one pump (132) configured to pump the liquid medium from a reservoir (4) to the inlet (102) of the module (10).

13. The system (1) of claim **12,** comprising at least two modules (10), wherein the at least two modules (10) are connected in series or in parallel.

14. A method for incubating a biological material in a module (10) according to any one of claims **1** to **10,** the method comprising a step of conveying the liquid medium through the at least one incubating chamber (11) via the perfusion system (12).

15. The method of claim **14,** wherein the liquid medium is conveyed at a continuous flow rate or a pulsatile flow rate.
